(19) **Europäisches Patentamt / European Patent Office / Office européen des brevets**

(11) **EP 3 537 157 B1**

(12) **FASCICULE DE BREVET EUROPEEN**

(45) Date de publication et mention de la délivrance du brevet:
**19.01.2022 Bulletin 2022/03**

(21) Numéro de dépôt: **19160446.1**

(22) Date de dépôt: **04.03.2019**

(51) Classification Internationale des Brevets (IPC):
*A01C 21/00* (2006.01)  *G01N 33/24* (2006.01)

(52) Classification Coopérative des Brevets (CPC):
**A01C 21/007;** G01N 2033/243; G01N 2033/245

(54) **PROCÉDÉ POUR L'OBTENTION D'UN INDICATEUR ÉLÉMENTAIRE DE LA RÉSILIENCE DE POPULATIONS BACTÉRIENNES ET AZOTOBACTÉRIENNES DU SOL ET SON APPLICATION À LA FERTILISATION RAISONNÉE**

VERFAHREN ZUR ERLANGUNG EINES ELEMENTAREN INDIKATORS DER WIDERSTANDSFÄHIGKEIT VON BAKTERIELLEN UND AZOTOBAKTERIELLEN POPULATIONEN UND SEINE ANWENDUNG AUF AUSGEWOGENE DÜNGUNG

METHOD FOR OBTAINING AN ELEMENTARY INDICATOR OF THE RESILIENCE OF BACTERIAL AND AZOTOBACTERIAL SOIL POPULATIONS AND ITS APPLICATION TO RATIONED FERTILIZATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.03.2018 FR 1870241**

(43) Date de publication de la demande:
**11.09.2019 Bulletin 2019/37**

(73) Titulaire: **Polyor SARL**
**54000 Nancy (FR)**

(72) Inventeur: **CLAUDE, Pierre-Philippe**
**54000 Nancy (FR)**

(56) Documents cités:
EP-A1- 2 730 926    EP-A1- 2 845 906
EP-A1- 2 942 621    EP-A1- 3 120 679
EP-A1- 3 335 536    US-A- 5 668 719
US-A1- 2012 014 748    US-A1- 2013 046 468

Remarques:
Le dossier contient des informations techniques présentées postérieurement au dépôt de la demande et ne figurant pas dans le présent fascicule.

**Description**

**DOMAINE TECHNIQUE DE L'INVENTION**

Analyses biologique et/ou physico-chimique des sols arables, et fertilisation raisonnée des cultures agronomiques. Accessoirement, il est aussi question de microbiologie des sols et de biofertilisation par inoculation azotobactérienne.

**ÉTAT DE LA TECHNIQUE**

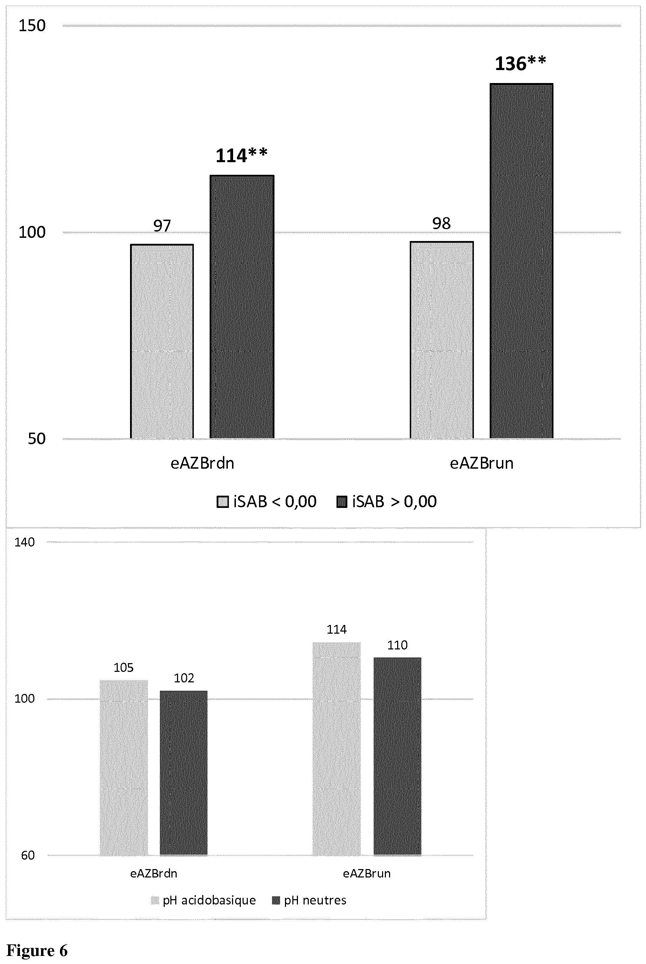

Bien qu'a priori les pH plutôt neutres (6,5 à 7,5) soient favorables aux azotobactéries, et plus particulièrement aux *Azotobacteraceae* (eg. Martyniuk et Martyniuk 2003), ils ne favorisent pas nécessairement l'efficacité de la fertilisation azotobactérienne (Claude et Fillion 2004). Au contraire (voir par exemple ici, Figure 6). Celle-ci dépend plutôt de la résilience acidobasique des populations azotobactériennes indigènes (cf. EP2730926). Plus les populations indigènes d'azotobactéries sont résilientes, moins l'azotobactérisation de la résidusphère, y compris par inoculation, aura d'effets sur les rendements agronomiques.

Une précédente invention (EP2730926) permet d'apprécier cette résilience acidobasique des azotobactéries indigènes confrontées à des variations intra-saisonnières de pH à hauteur de l'acidité échangeable du sol, soit approximativement la différence $pH_{eau}$ - $pH_{KCl}$, ou $\Delta pH$ (lire « delta pH »). Cependant, EP2730926 ne décrit pas comment *quantifier* précisément cette résilience. En effet, EP2730926 ne propose que de superposer des courbes dites fixes et variables comprenant un intervalle de confiance (ic), et de graduer la résilience azotobactérienne par rapport à l'angle, $\theta$, entre ces courbes fixes et variables. Si la courbe dite variable s'écarte significativement de la courbe fixe en raison d'un angle, $\theta$, important, les populations bactériennes indigènes sont dites peu ou pas résilientes. Le cas échéant, le renforcement de ces populations par azotobactérisation des résidus de culture est préconisé. Rien de plus.

Or ce $\Delta pH$ affecte bel et bien *l'efficacité de l'azotobactérisation* (eAZB) des résidus de culture (Claude et Fillion 2004 ; EP2730926), mais en l'état est difficilement intégrable à titre indicateur élémentaire d'eAZB dans le cadre d'une véritable *fertilisation azotobactérienne raisonnée* (FAR ; EP17196251.7).

**DIVULGATION DE L'INVENTION**

*Problème technique*

Faute d'un indice élémentaire quantitatif, et malgré l'avancé que représente EP2730926, il est aujourd'hui difficile d'évaluer objectivement la résilience acidobasique des populations azotobactériennes indigènes d'un sol arable. En effet, EP2730926 ne propose pas véritablement un tel indicateur élémentaire facilement intégrable dans une démarche de *fertilisation azotobactérienne raisonnée* (FAR ; cf. EP17196251.7).

*Solution technique*

La solution technique proposée consiste à quantifier cette résilience à l'aide d'un indicateur élémentaire (iSAB), et plus facilement obtenable que l'indice déjà proposé dans EP2730926. Concrètement, il s'agit d'une méthode de fertilisation raisonnée adaptée à l'azotobactérisation (AZB) des résidus de culture cellulosiques au sol (RCS) et de cultures intermédiaires pièges à nitrates (cipan) comportant un indicateur élémentaire de la résilience de la fraction bactérienne de la microflore d'un sol arable en termes de diversité, d'activité et/ou d'abondance lorsque confrontée à des variations de pH du sol environnant ne nécessitant *plus (pas)* (a) l'échantillonnage de chaque parcelle agricole, (b) la mesure de la dynamique de la fraction bactérienne du sol en termes de diversité, d'activité et/ou d'abondance à différentes valeurs de pH correspondant à l'étendue de l'acidité échangeable et/ou (c) la comparaison de cette mesure de la dynamique bactérienne avec des valeurs optimales pour ce sol arable, sachant que ;

la mesure de la résilience est obtenue par superposition de la courbe décrivant cette variation de la dynamique de ladite fraction bactérienne de la microflore du sol en fonction des susdites différentes valeurs de pH imposées *in vitro* (courbe variable), à une autre courbe fixée horizontalement (courbe fixe) à partir d'un pH optimal pour ladite dynamique bactérienne en termes de diversité, d'activité et/ou d'abondance, relative ou absolue,

mais plutôt caractérisée en ce que l'indicateur élémentaire, iSAB, est obtenu directement à l'aide de relations préétablies, à savoir ;

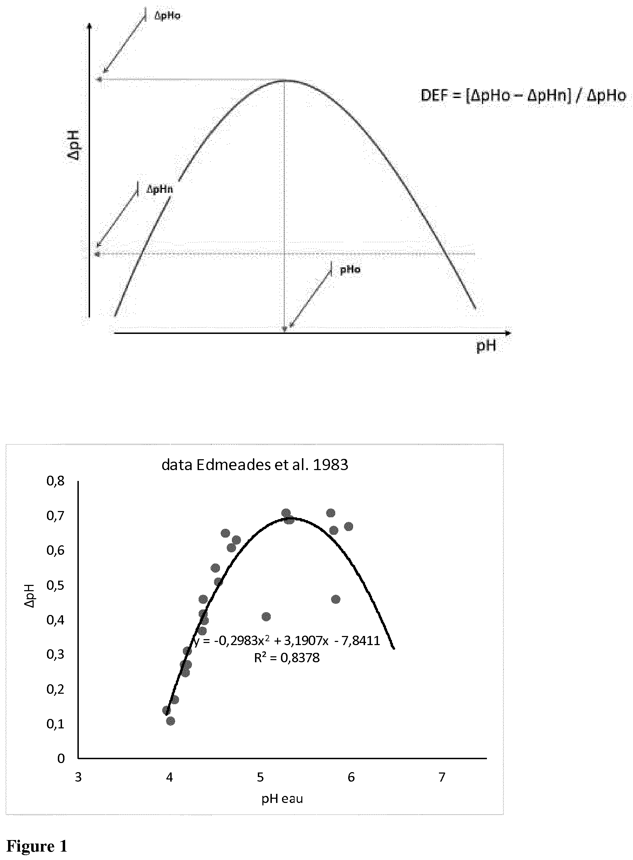

- une première relation [$\Delta pH :: pH$] représentant quantitativement la *défiance* acidobasique du sol, DEF, définie comme le ratio entre l'amplitude de l'acidité échangeable ($\Delta pHo$ - $\Delta pHn$) et le $\Delta pH$ (Figure 1) associée au pHo ($\Delta pHo$) obtenu par la dérivée première égale à 0,00 pour la susdite relation [$\Delta pH :: pH$] (Figure 1),

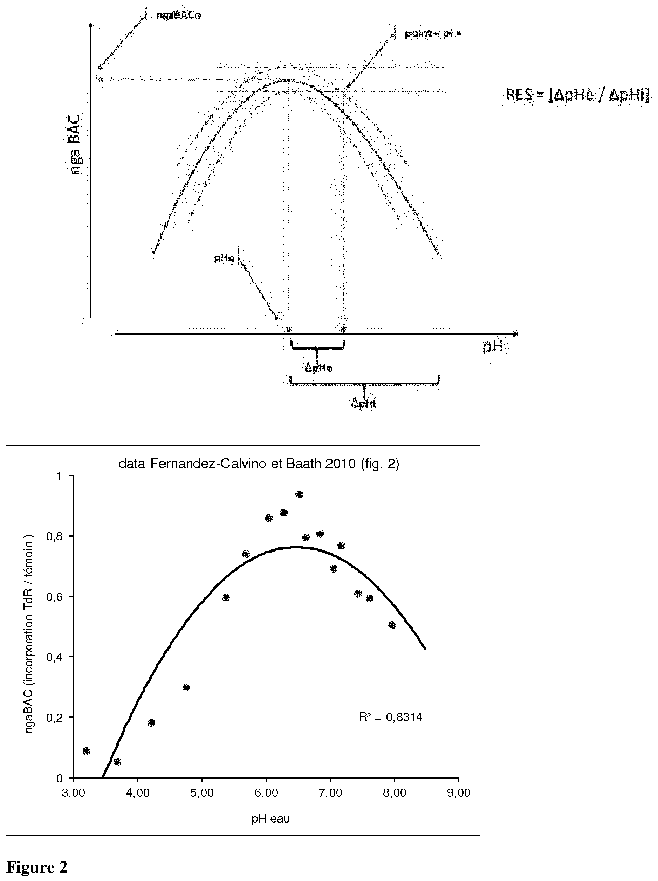

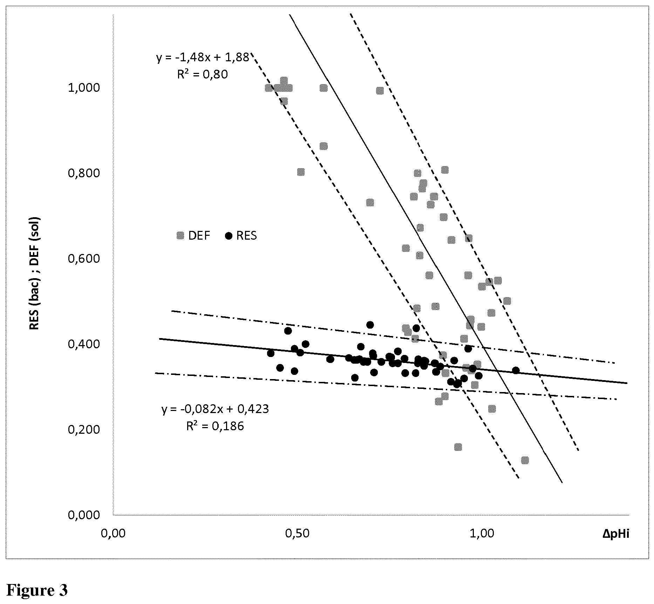

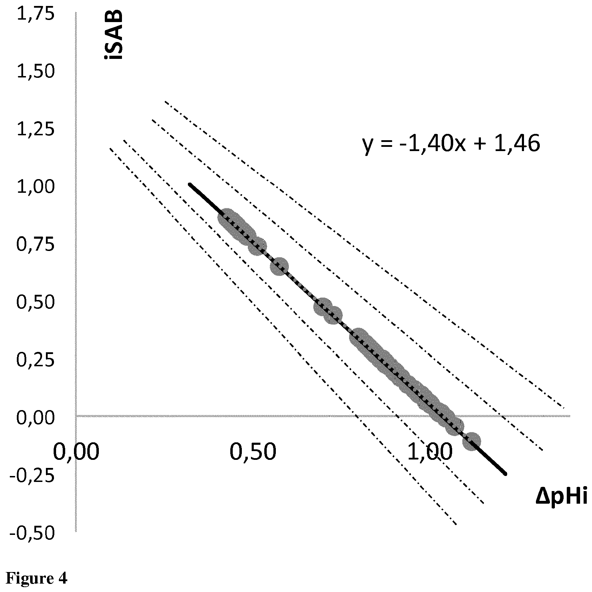

- une deuxième relation [ngaBAC :: pH] représentant quantitativement la *résilience* acidobasique des azotobactéries indigènes du sol, RES, définie comme le ratio entre le $\Delta pH$ effectif ($\Delta pHe$) et $\Delta pHi$ (Figure 2), $\Delta pHe$ étant repérable dès que ngaBAC est appréciablement affectée par $\Delta pH$ lorsque le minimum d'une certaine *l'intervalle de confiance* (ic) de la susdite courbe fixe croise (intersecte) le maximum de l'ic de la susdite courbe variable au point « pi »,
- la superposition, graphique ou mathématique, par rapport à $\Delta pHi = [pH_{eau} - pH_{KCl}]$, de ces deux relations - DEF et RES (Figure 3), afin d'obtenir la différence iSAB = [DEF - RES] (Figure 4) à titre d'indicateur élémentaire du degré de résilience acidobasique des populations azotobactériennes indigènes du sol,
- et enfin l'intégration d'iSAB dans un indice agrégé d'eAZB (Figures 5 et 6) servant au calcul des doses prévisionnelles d'engrais azotés (dN) adaptées au fonctionnement de telles azotobactérisation des résidus de culture cellulosiques en fertilisation raisonnée (Figure 7).

La première relation [$\Delta pH$ :: pH] représentant quantitativement la *défiance* acidobasique du sol, DEF, est obtenue préalablement à l'aide de valeurs pH du sol, avantageusement le $pH_{eau}$ afin d'assurer un $\Delta pH$ mesurable ($\Delta pHi$), prélevées à travers le temps ou résultant de différentes pratiques culturales (eg. Edmeades et al. 1983), ou encore provenant de variations spatiales répertoriées dans diverses bases de données existantes (eg. BDAT (INRA-GisSol 2018), NCSS (NCSS 2018)). DEF est exprimée par rapport à $\Delta pHi$ selon la relation DEF = $a_1 + b_1 \cdot \Delta pHi$ (Figure 3), sachant qu'à ce stade les coefficients $a_1$ et $b_1$ peuvent appartenir à une gamme de valeurs comprises entre (Tableau 2) 1,60 et 2,00 pour $a_1$ et -1,15 et -1,65 pour $b_1$.

La deuxième relation, [ngaBAC :: pH] représentant quantitativement la *résilience* acidobasique des azotobactéries indigènes du sol, RES, lesdites intervalles de confiance (ic) sont fonctions des variations des valeurs de pH et de la dynamique des population azotobactérienne (ngaBAC) correspondante selon l'équation tirée de Wonnacott et Wonnacott (1991, éq. 12-13, p. 438) ;

$$ic = (a + bX_o) +/- t_{0,025} \cdot s \cdot racine(1/n + [(X_o - \dot{X})^2 / \sum X^2])$$

lorsque que $(a + bX_o)$ décrit empiriquement la progression d'ngaBAC selon $X_o$ = pH à partir de pHo (voir les deux droites divergentes aux Figures 2, 3 et 4), $t_{0,025}$ est la valeur critique de la statistique t lorsque les ddl (degrés de liberté) = nombre d'observations moins 1, s est *l'écart-type* et $(X_o - \dot{X})^2$ la somme des carrés des différences entre $X_o$ et la moyenne $\dot{X}$ pour les n observations. RES est exprimée par rapport à $\Delta pHi$ selon la relation RES = $a_2 + b_2 \cdot \Delta pHi$ (Figure 3), sachant qu'à ce stade les coefficients $a_2$ et $b_2$ peuvent appartenir à une gamme de valeurs comprises entre (Tableau 2) 0,003 et 0,027 pour $a_2$ et 0,315 et 0,365 pour $b_2$.

La superposition, graphique ou mathématique, par rapport à $\Delta pHi = [pH_{eau} - pH_{KCl}]$, de ces deux relations - DEF et RES (Figure 3), afin d'obtenir la différence iSAB = [DEF - RES] (Figure 4) à titre d'indicateur élémentaire du degré de résilience acidobasique des populations azotobactériennes indigènes du sol donne lieu à une famille de relations iSAB fonctions de $\Delta pHi$ selon le type de situations agronomiques, types de sols, localisation géographique de la parcelle ou toutes autres caractéristiques agro-pédo-climatiques pouvant affecter le statut acidobasique (SAB) du sol. iSAB, est exprimé par rapport à $\Delta pHi$ selon la relation iSAB = $a_3 + b_3 \cdot \Delta pHi$ (Figure 4), sachant qu'à ce stade les coefficients $a_3$ et $b_3$ p'ouvent appartenir à une gamme de valeurs comprises entres (Tableau 2) 1,75 et 1,85 pour $a_3$ et -1,72 et -1,80 pour $b_3$.

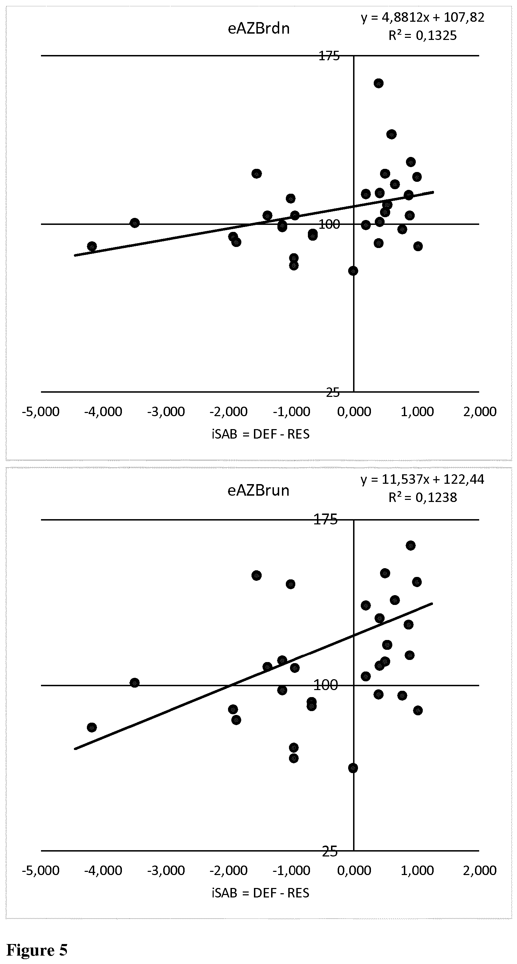

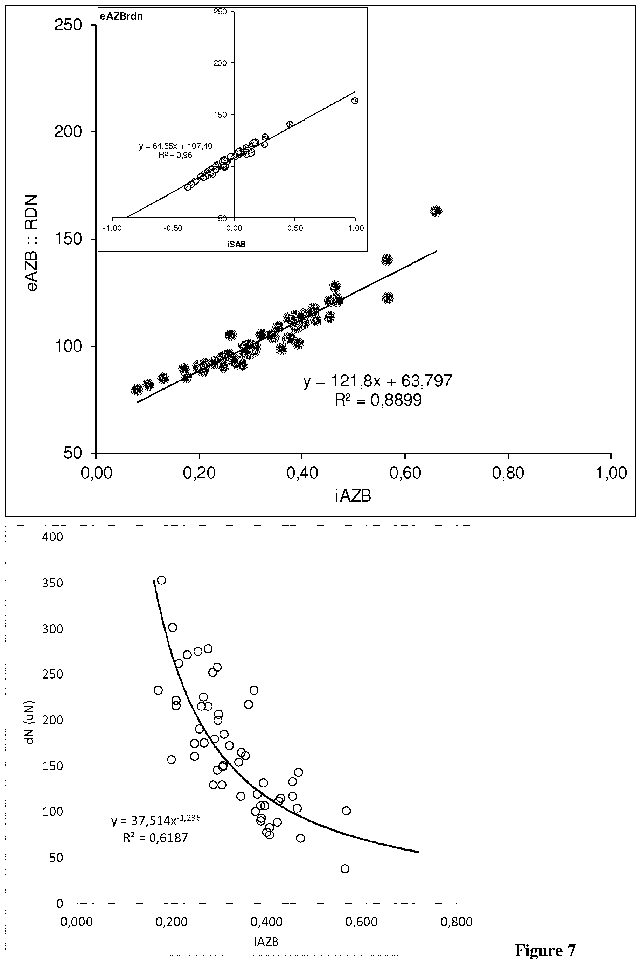

Enfin, l'intégration d'iSAB dans un indice agrégé d'eAZB (Figures 5 et 6) servant au calcul des doses prévisionnelles d'engrais azotés (dN) adaptées à l'azotobactérisation des résidus de culture (Figure 7) ce fait comme suit (cf. EP17196251.7) ;

$$iAZB = \sum(Si \times Pi) \text{ pour les } i = 1, 2, 3 \ldots \rightarrow \text{nombre d'indicateurs } (n),$$

y compris iSAB

*Si* étant ici le score normalisé (base 100 ou 1,00) de l'indicateur élémentaire affectant eAZB, et *Pi* le poids (0,00 à 1,00) attribué à ce score, pAZB est ici en sorte la contribution (fourniture) en N des microorganismes phytogènes du sol, y compris ceux réintroduits par l'inoculation, et en ce que dN fonction de l'indicateur agrégé, iAZB, obtenu par agrégation des indicateurs élémentaires d'eAZB est calculé comme suite ;

$$dN = a \times pRDT - pAZB$$

sachant que ;

- $$a = b_{ARVALIS} / eAZB_{RUN}$$

- $$pAZB = pRDN - pRDN/eAZB_{RDN}$$

et que pRDT et pRDN sont les objectifs, ou potentiels, de rendements agronomiques ($Qx_{grain}$/ha) et protéiques (kg-$N_{grain}$/ha), $eAZB_{RDN}$ et $eAZB_{RUN}$ les valeurs d'eAZB en termes de RDN et RUN (rendements unitaires ; kg-$N_{grain}$ ou kg-$N_{biomasse}$ /unité d'N fertilisant), respectivement et par rapport à un témoin non traité, et que a est le coefficient b dit d'Arvalis™ ($b_{ARVALiS}$), alias « besoin unitaire » (i.e. unités d'N par $Qx_{grain}$), divisée par $eAZB_{RUN}$.

*Avantages apportés*

De simples valeurs $\Delta pHi = [pH_{eau} - {}_pH_{KCL}]$ peuvent maintenant estimer directement et quantitativement cette résilience acidobasique des azotobactéries indigènes du sol. L'utilisateur n'a plus à effectuer lui-même le titrage de l'acidité échangeable proposé dans EP2730926 pour chaque sol à analyser ; il peut utiliser directement et dès maintenant les relations DEF, RES et iSAB fonctions de dpHi proposées ici.

Sigles et définitions ;

**Dynamique** des populations bactériennes (**ngaBAC;** EP2730926**)** ; changements constatés à travers le temps et/ou en fonction d'une variation des paramètres physico-chimiques (eg. pH) du **n**ombre de cellules bactériennes (n), leurs masses et/ou leurs abondances relative à celles des autres fractions de la microflore du sol (**g**), leurs diversité génomique et/ou phénotypiques et, surtout, leurs activités métaboliques et/ou cataboliques (**a**).

**Fraction bactérienne** (**BAC**) ; biomasse bactérienne du sol séparée chimiquement ou physiquement (eg. par centrifugation) d'une suspension aqueuse du sol (cf. Bakken 1985).

**Résilience acidobasique** ; caractéristique des bactéries du sol capables de maintenir une certaine dynamique optimale en dépit de variations de pH à hauteur de $\Delta pH$.

**pHo** (sols ; Figure 1) : pH du sol lorsque $\Delta pH$ est optimal selon la relation [$\Delta pH :: pH$]. pHo est pH tellurique auquel $\Delta pH$ est le plus important, apportant ainsi aux azotobactéries du sol une certain « zone de confort » plus ample et plus difficilement comblée à travers le temps ou selon les pratiques culturales.

**pHo** (bactéries ; Figure 2) : pH du sol lorsque ngaBAC est optimal (ngaBACo ; Figure 2) selon la relation bimodale ngaBAC :: pH. pHo est ici une mesure de l'acidité à laquelle la dynamique des populations bactériennes du sol (ngaBAC) est la plus grande, voire optimale.

**$pH_{eau}$, $pH_{KCl}$, $pH_{CaCl2}$** : Mesure de l'acidité du sol en terme de pH à l'aide d'une solution d'extraction strictement aqueuse (eau), ou saline comportant différentes concentrations de KCl ou de $CaCl_2$ selon le cas.

**$\Delta pH$** (*alias* **acidité échangeable**) : acidité du sol attribuable à l'aluminium et l'hydrogène adsorbés sur le complexe d'échange du sol, et échangeables par d'autres cations tels que le $K^+$ et/ou le $Ca^{++}$.

**$\Delta pHi$** : $\Delta pH$ moyen d'un ensemble de $\Delta pH$ sur la base de la différence $pH_{eau} - pH_{KCl}$ (ou $pH_{CaCl2}$) mesuré par l'utilisateur de la présente invention.

**$\Delta pHo$** : $\Delta pH$ optimal correspondant à la première dérivée nulle (0,00) de la relation [$\Delta pH :: pH$]. $\Delta pHo$ est ici la valeur de $\Delta pH$ la plus vraisemblable ; c'est **$\Delta pHo$** qui servira donc au calcul de DEF.

**$\Delta pHn$** : $\Delta pH$ minimum d'un ensemble de $\Delta pH$.

**DEF** (Figure 1) : « défiance » acidobasique d'un sol à l'égards de populations azotobactériennes qu'il abrite. Formellement DEF = $[\Delta pHo - \Delta pHn] / \Delta pHo$, i.e. l'amplitude par rapport à la valeur minimum de l'acidité échangeable (correspondant à pHo) observée in situ à travers le temps, selon les pratiques culturales, etc.

**RES** (Figure 2) : résilience acidobasique des azotobactéries indigènes du sol confrontées à des variations de pH à hauteur de $\Delta pH = [pHeau - pH_{KCl}]$ voire $[pHeau - pH_{CaCl2}]$.

**iSAB** (Figures 3, 4 et 5) : *indicateur élémentaire* (Lairez et al. 2015) de la résilience acidobasique des azotobactéries indigènes telluriques défiées par $\Delta pH$. Formellement ; iSAB = [DEF - RES].

**eAZBrdn** : efficacité relative en termes de *rendement protéique* (RDN ; kg-$N_{grain}$/ha) de l'azotobactérisation des résidus de culture (cellulosiques) au sol (RCS) par rapport à un témoin non azotobactérisés

**eAZBrun** : efficacité relative en termes de *rendement unitaire* (RUN ; kg-$N_{grain}$/uN) de l'azotobactérisation des résidus de culture (cellulosiques) au sol (RCS) par rapport à un témoin non azotobactérisés

**iAZB** (EP17196251.7) : *indicateur agrégé* (Lairez et al. 2015) d'indicateurs élémentaires d'eAZBrdn et eAZBrun, tel qu'iSAB par exemple.

**dX** (Comifer 2013) : dose prévisionnelle d'engrais azotés pour cultures agronomiques calculée selon la *méthode du bilan* (Colomb 2017) exprimée en unités d'N (uN ; kg-$N_{fertilisant}$/ha).

**dN** (Claude 2017) : dose prévisionnelle d'engrais azotés pour cultures agronomiques calculée selon la *fertilisation azotobactérienne raisonnée* (FAR), généralement exprimée en unités d'N (uN ; kg-$N_{fertilisant}$/ha).

**FAR** (Claude 2017) : méthode de fertilisation raisonnée comportant un ajustement fonction d'eAZB des *besoins unitaires* (*cf.* coefficient $b_{Arvalis}$) et des fournitures en N provenant des sols dans lesquels sont enfouis des résidus de culture azotobactérisés, y compris par inoculation. Les doses prévisionnelles d'engrais-N (dN) ainsi calculées sont ainsi expressément adaptées à de telles azotobactérisations.

**BRÈVES DESCRIPTION DES FIGURES**

**Figure 1** : Relation bimodale [ΔpH :: pH], avantageusement ici quadratique de type ΔpH = a·(pH)$^2$ + b·(pH) + c, et calcul de DEF. ΔpH (pHeau - $pH_{KCl}$) n'est fonction linéaire de pH. Au contraire, les pH marginaux (acidiques ou basiques) par rapport à une certaine valeur centrale (pHo) sont les plus faibles dénotant une quantité acidité échangeable plus restreinte et plus sujette à être appréciablement, voire complètement réalisée à travers la campagne. Le cas échéant, les azotobactéries du sol devront affronter ces variations de pH capables d'affecter leur nombre (n), masse (g) et/ou activité (a), d'où la notion de « ngaBAC » (Figure 2). DEF est définie comme suit ; [ΔpHo - ΔpHn] / ΔpHo, **ΔpHn** étant le ΔpH minimum dans l'ensemble des pH. (bas) A titre d'exemple, les données de Edmeades et al. 1983.

**Figure 2 :** Relation bimodale entre ngaBAC et pH du sol et calcul de RES. ngaBAC est lui aussi bimodal par rapport à pH. La mesure de la résilience est obtenue par superposition de la courbe décrivant cette variation de la dynamique de ladite fraction bactérienne de la microflore du sol en fonction des susdites différentes valeurs de pH imposées *in vitro* (courbe variable), à une autre courbe fixée horizontalement (courbe fixe) à partir d'un pH optimal (pHo) et/ou supposé optimal pour ladite dynamique bactérienne en termes de diversité, d'activité et/ou d'abondance, relative ou absolue. Cette caractéristique des azotobactéries est explicitée dans EP2730926. ΔpHe peut maintenant être repéré par rapport à pHo (Figure 1) comme la portion de ΔpHi = $pH_{eau}$ - $pH_{KCl}$ n'affectant pas sensiblement ngaBAC. Quantitativement, cette *résilience* des azotobactéries du sol est repérable lorsque l'intervalle de confiance (ic ; *cf.* EP2730926) fixe inférieur rejoint l'intervalle de confiance variable supérieur au point « pi ». ΔpHe est donc le degré de réalisation de l'acidité échangeable que peuvent subir les azotobactéries du sol sans réduction de ngaBAC. RES est définie comme suit ; RES = ΔpHe / ΔpHi. (bas). A titre d'exemple, les données de Fernadez-Calvino et Baath 2010 (*cf.* EP2730926).

**Figure 3** : Mise en relation de DEF et RES par rapport à ΔpHi. DEF et RES sont mis en relations par rapport à ΔpHi conjointement afin de d'obtenir iSAB = DEF - RES. La pression ΔpH, ou le choc de la variation acido-basique provenant de la réalisation de l'acidité échangeable à travers la campagne sera donc d'autant plus faible que ΔpHi est important (Figure 4). En principe, l'efficacité de l'azotobactérisation (eAZB) des résidusphères, y compris et notamment par inoculation des résidus de culture au sol (RCS), sera d'autant plus grande si cette pression ΔpH est importante puisque les populations azotobactériennes indigènes profiteront ainsi le plus de tels renforts. Sans cette pression acido-basique, les populations indigènes peu affectées nécessiteront peu ou pas de telles augmentations de ngaBAC. iSAB reflète donc l'opportunité d'augmenter les contingents d'azotobactéries résidusphériques en vue d'une augmentation d'eAZB (Figure 5). A noter enfin que ces relations [DEF :: ΔpHi] et [RES :: ΔpHi] peuvent être ajustés selon la situation agronomique, le lieu (eg. les départements français), le type de sol (eg. argilo-calcaire, etc.). J'ai indiqué ces éventuelles variantes en pointillés.

**Figure 4 :** Illustration de la progression d'iSAB par rapport à ΔpHi. iSAB étant défini comme la différence DEF - RES (Figure 3), cet indicateur élémentaire d'eAZB décroit progressivement en fonction de ΔpHi favorisant ainsi l'accroissement d'eAZB (Figures 5 et 6). A noter qu'étant donné d'éventuels ajustements agro, pédo-climatiques des fonctions (relations) [DEF :: ΔpHi] et [RES :: ΔpHi] (*cf.* Figure 3), la relation [iSAB :: ΔpHi] sera elle aussi raffinée en ce sens (*cf.* pointillés) et donnera lieu à une famille de relations iSAB fonction de ΔpHi plus particulières et précises.

**Figure 5** : Illustration de la progression d'eAZBrdn et d'eAZBrun par rapport à iSAB sur la base de données agronomiques existantes déjà mentionnées dans Claude 2017 et EP17196251.7).

**Figure 6** : Comparaison d'eAZB pour des iSAB négatifs de positifs (fonction t.test() ; Excel™). A ce stade, la relation entre eAZB et iSAB (Figure 5) est nécessairement très lâche puisque iSAB n'est qu'un des multiples indicateurs élémentaires d'eAZB (cf. EP2728353, EP2845906, EP2942621, etc.). Cela dit, les valeurs d'eAZBrdn et eAZBrun associées à des iSAB négatifs (i.e. RES > DEF) sont en moyenne nettement inférieures à celles associées à des iSAB positifs (i.e. RES < DEF). Au contraire, les pH neutres (disons 6,5 à 7,5) a priori favorables aux *Azotobacteraceae* telluriques indigènes ne sont vraisemblablement pas favorables à l'augmentation d'eAZB. Au contraire.

**Figure 7** : Relation d'eAZB et l'indice agrégé iAZB comprenant entre autres iSAB à titre d'indicateur élémentaire de la résilience acidobasique des azotobactéries indigènes. A partir de données agro-pédo-climatiques de 30 essais agronomiques de blés de de colza (2011 à 2015), des indices élémentaires d'eAZB tels qu'iSAB peuvent ainsi être agrégés par analyse multicritère en un indice composé d'eAZB, iAZB (EP17196251.7). iSAB corrélé à eAZB (en abîme) contribue donc à calibrer la dose d'engrais-N (dN). A titre d'illustration (bas), iAZB intégrant iSAB est ici bel et bien inversement corrélé avec dN ; i.e. pour un potentiel de rendement donné, plus cet indicateur agrégé d'eAZB est important, plus dN sera faible.

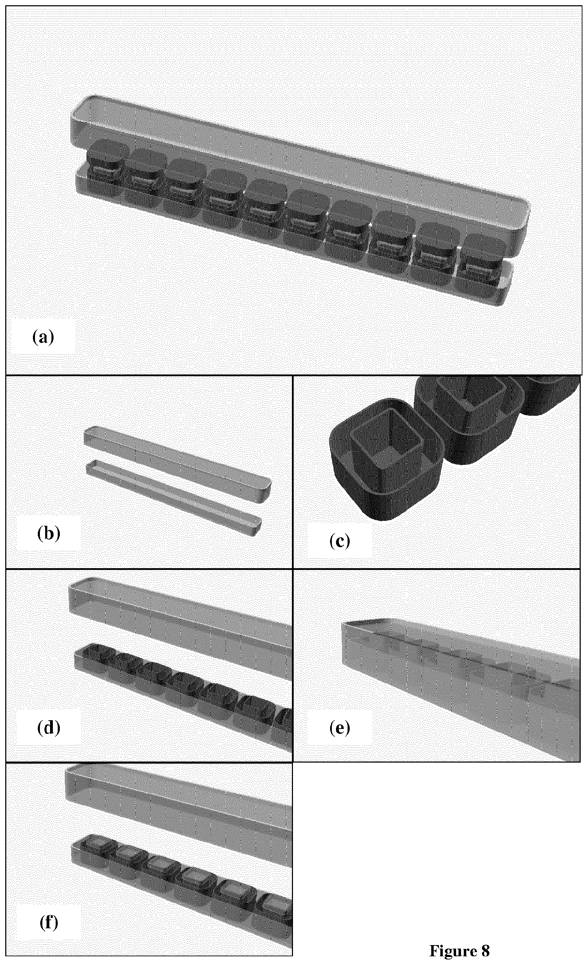

**Figure 8** : Dispositif permettant d'évaluer simultanément iSAB pour les bactéries et les azotobactéries du sol. (a) Vue d'ensemble avec couvercle surplombant. (b) Boîtier abritant les puits bicoques. (c) Vue rapprochée d'un des puits bicoques ; il y en a dix (10) proposés ici, mais ce nombre est au choix. (d) Les puits sont en principe amovibles, mais peuvent aussi être solidaire avec le fond du boîtier. (e) Le couvercle protecteur peut servir à protéger les puits pendant l'incubation. (f) S'il est question d'azotobactéries anaérobiques, il est possible de recouvrir hermétiquement l'un (ici) ou les deux compartiments des puits bicoques.

## MODE DE RÉALISATION DE L'INVENTION

Les fonctions [DEF :: ΔpHi] et [RES :: ΔpHi] (Figure 3) sont préétablies sur la base de données expérimentales, y compris parmi celles déjà publiées. La fonction iSAB = [DEF - RES] :: ΔpHi (Figure 4) directement applicable par l'utilisateur lui permet d'obtenir iSAB à partir de simples valeurs de dpHi. Cette fonction sera raffinée et précisée selon les lieux, situations agronomiques, types de sols, pratiques culturales, etc. dans le cadre d'un développement expérimental continu.

iSAB = [DEF - RES] et par la suite mis en relation avec eAZB. DEF est obtenu en traçant une série de fonctions bimodales (Figure 1) de type [ΔpH :: pH] ($pH_{eau}$, $pH_{KCL}$ ou $pH_{CaCl2}$, au choix). Les coefficients a, b et c de ces fonctions, avantageusement quadratiques, sont variables selon la situation agro-pédo-climatique (Tableau 1).

**Tableau 1** : Quelques données utilisées pour le calcul des valeurs de pHo, ΔpHo, ΔpHn et ΔpHi afin d'obtenir DEF et iSAB. Les coefficients des fonctions de type $\Delta pH = a(pH)^2 + b(pH) + c$ (Figure 1) sont rapportés. ΔpHo et pHo sont définis par la première dérivée nulle (0,00). Les données BDAT proviennent de INRA-GisSol 2018, et celles notées NCSS de NCSS 2018. Certaines données BDAT et NCSS ont été réparties en *agro-pédo-climats* (iAPC ; EP2728353), d'autre proviennent de la région Centre en France (BDAT). iSAB est la différence entre DEF et RES ([DEF - RES]$_{\Delta pHi}$ ; Figure 3) pour chacune des valeurs de ΔpHi.

| Jeu de données (n OBS) | n OBS | pHo | ΔpHi | ΔpHo | ΔpHn | DEF | a | b | c | iSAB |
|---|---|---|---|---|---|---|---|---|---|---|
| *Edmeades et al. 1983* | 24 | 0,69 | 0,463 | 3,234 | 0,1100 | 101,6% | -2,2983 | 3,1907 | -7,8411 | 1,0918 |
| *Martyniuk et al. 2003* | 30 | 5,58 | 0,572 | 0,732 | 0,0000 | 100,0% | -0,1121 | 1,2515 | -2,7605 | 0,8622 |
| *Fernandez-Calvino et al. 2010* | 7 | 5,61 | 0,799 | 0,946 | 0,5400 | 42,9% | -0,4720 | 5,291 | -13,891 | 0,4616 |
| *iAPC1 / BDAT* | 4 | | | | | | | | | |
| *iAPC2 / BDAT* | 37 | 5,96 | 0,825 | 0,911 | 0,4700 | 48,4% | -0,0715 | 0,8527 | -1,6316 | 0,3292 |
| *iAPC3 / BDAT* | 95 | | | | | | | | | |
| *iAPC 4 / BDAT* | 142 | 4,93 | 0,858 | 0,958 | 0,4200 | 56,2% | -0,0229 | 0,2256 | 0,4027 | 0,2597 |

(suite)

| Jeu de données (n OBS) | n OBS | pHo | ΔpHi | ΔpHo | ΔpHn | DEF | a | b | c | iSAB |
|---|---|---|---|---|---|---|---|---|---|---|
| iAPC 5 / BDAT | 201 | 6,32 | 0,871 | 0,943 | 0,2400 | 74,5% | -0,0845 | 1,0677 | -2,43 | 0,2323 |
| iAPC 6 / BDAT | 176 | 3,54 | 0,839 | 1,018 | 0,2400 | 76,4% | -0,0154 | 0,109 | 0,8248 | 0,2997 |
| iAPC 7 / BDAT | 185 | 4,52 | 0,842 | 0,941 | 0,2100 | 77,7% | -0,0151 | 0,1365 | 0,6328 | 0,2934 |
| iAPC 8 / BDAT | 138 | 5,44 | 0,815 | 0,900 | 0,2300 | 74,4% | -0,0278 | 0,3027 | 0,0758 | 0,3503 |
| iAPC 9 / BDAT | 122 | | | | | | | | | |
| iAPC 10 / BDAT | 100 | 7,42 | 0,826 | 1,802 | 0,3600 | 80,0% | -0,006 | 0,089 | 1,4722 | 0,3271 |
| iAPC 11 / BDAT | 72 | 4,70 | 0,862 | 1,025 | 0,2800 | 72,7% | -0,021 | 0,1974 | 0,5609 | 0,2513 |
| iAPC 12 / BDAT | 80 | 4,70 | 0,896 | 1,025 | 0,3100 | 69,7% | -0,021 | 0,1974 | 0,5609 | 0,1797 |
| iAPC 13 / BDAT | 56 | | | | | | | | | |
| iAPC 14 / BDAT | 35 | 6,58 | 0,902 | 0,930 | 0,6200 | 33,3% | -0,0528 | 0,6951 | -1,3582 | 0,1670 |
| iAPC 15 / BDAT | 54 | 6,52 | 0,833 | 0,886 | 0,2900 | 67,3% | -0,1163 | 1,5172 | -4,0619 | 0,3124 |
| région Centre 1 | 33 | 7,15 | 1,020 | 1,168 | 0,530 | 54,6% | -0,449 | 6,418 | -21,779 | 0,0159 |
| région Centre 2 | 33 | 6,97 | 0,895 | 0,943 | 0,590 | 37,4% | -0,099 | 1,386 | -3,888 | 0,2399 |
| région Centre 3 | 33 | 6,93 | 0,964 | 1,104 | 0,390 | 64,7% | -0,230 | 3,189 | -9,953 | 0,1162 |
| région Centre 4 | 33 | 6,90 | 0,988 | 1,128 | 0,730 | 35,3% | -0,259 | 3,575 | -11,213 | 0,0732 |
| région Centre 5 | 33 | 6,70 | 0,971 | 1,028 | 0,680 | 33,8% | -0,103 | 1,387 | -3,620 | 0,1037 |
| région Centre 6 | 33 | 6,97 | 1,044 | 1,196 | 0,540 | 54,8% | -0,337 | 4,692 | -15,153 | -0,0271 |
| région Centre 7 | 33 | 7,16 | 1,068 | 1,282 | 0,640 | 50,1% | -0,512 | 7,324 | -24,922 | -0,0702 |
| région Centre 8 | 33 | 6,95 | 0,958 | 1,161 | 0,760 | 34,5% | -0,423 | 5,875 | -19,255 | 0,1270 |
| région Centre 9 | 33 | 7,20 | 0,970 | 0,958 | 0,520 | 45,7% | -0,112 | 1,615 | -4,858 | 0,1055 |
| région Centre 10 | 33 | 6,91 | 0,917 | 1,037 | 0,370 | 64,3% | -0,248 | 3,427 | -10,804 | 0,2004 |
| région Centre 11 | 33 | 7,16 | 1,025 | 1,139 | 0,600 | 47,3% | -0,175 | 2,509 | -7,845 | 0,0069 |
| région Centre 12 | 33 | 6,76 | 1,000 | 1,162 | 0,540 | 53,5% | -0,248 | 3,355 | -10,174 | 0,0517 |
| iAPC 1 / NCSS | 320 | 6,68 | 0,442 | 0,512 | -0,200 | 139,0% | -0,028 | 0,374 | -0,739 | 1,1360 |
| iAPC 2 / NCSS | 305 | 6,22 | 0,472 | 0,547 | -0,500 | 191,4% | -0,050 | 0,625 | -1,398 | 1,0728 |

(suite)

| Jeu de données (n OBS) | n OBS | pHo | $\Delta$pHi | $\Delta$pHo | $\Delta$pHn | DEF | a | b | c | iSAB |
|---|---|---|---|---|---|---|---|---|---|---|
| iAPC 3 / NCSS | 272 | 6,30 | 0,449 | 0,557 | -0,100 | 117,9% | -0,059 | 0,739 | -1,772 | 1,1212 |
| iAPC 4 / NCSS | 303 | 6,30 | 0,453 | 0,527 | -0,200 | 137,9% | -0,044 | 0,548 | -1,200 | 1,1128 |
| iAPC 5 / NCSS | 250 | 6,40 | 0,464 | 0,551 | -0,300 | 154,5% | -0,052 | 0,666 | -1,582 | 1,0897 |
| iAPC 6 / NCSS | 243 | 5,98 | 0,479 | 0,556 | -0,200 | 136,0% | -0,046 | 0,549 | -1,086 | 1,0581 |
| iAPC 7 / NCSS | 248 | 6,05 | 0,438 | 0,489 | -0,200 | 140,9% | -0,035 | 0,423 | -0,791 | 1,1444 |
| iAPC 8 / NCSS | 228 | 6,39 | 0,431 | 0,526 | -0,200 | 138,0% | -0,068 | 0,872 | -2,259 | 1,1592 |
| iAPC 9 / NCSS | 209 | 6,36 | 0,422 | 0,517 | -0,600 | 216,0% | -0,062 | 0,785 | -1,981 | 1,1781 |
| iAPC 10 / NCSS | 221 | 6,28 | 0,450 | 0,521 | 0,000 | 100,0% | -0,066 | 0,828 | -2,079 | 1,1191 |
| iAPC 11 / NCSS | 181 | 6,05 | 0,476 | 2,208 | 0,000 | 100,0% | -0,037 | 0,451 | 0,842 | 1,0644 |
| iAPC 12 / NCSS | 214 | 6,24 | 0,423 | 0,463 | -0,300 | 164,8% | -0,033 | 0,410 | -0,817 | 1,1760 |
| iAPC 13 / NCSS | 167 | 5,83 | 0,428 | 0,496 | -0,100 | 120,2% | -0,053 | 0,613 | -1,290 | 1,1655 |
| iAPC 14 / NCSS | 157 | 6,15 | 0,435 | 0,505 | -0,100 | 119,8% | -0,056 | 0,692 | -1,622 | 1,1507 |
| iAPC 15 / NCSS | 116 | 5,83 | 0,458 | 0,525 | 0,000 | 100,0% | -0,050 | 0,580 | -1,165 | 1,1023 |
| iAPC 16 / NCSS | 113 | 5,60 | 0,446 | 0,529 | 0,000 | 100,0% | -0,051 | 0,566 | -1,056 | 1,1276 |
| iAPC 17 / NCSS | 92 | 6,08 | 0,466 | 0,535 | -0,100 | 118,7% | -0,058 | 0,702 | -1,600 | 1,0854 |
| iAPC 18 / NCSS | 69 | 5,74 | 0,422 | 0,482 | 0,000 | 100,0% | -0,033 | 0,383 | -0,617 | 1,1781 |

Chaque jeu de données permet de définir un couple $\Delta$pHo $\leftrightarrow$ pHo différents de $\Delta$pH minimum ($\Delta$pHn) pour chaque jeu de données. La fonction [DEF :: $\Delta$pHi] peut ainsi être tracée (Figure 3). Les données au Tableau 1 proviennent en partie d'études publiées (Edmeades et al. 1983, Martyniuk et al. 2003 et Fernandez-Calvino et al. 2010), et en partie de valeurs de pH$_{eau}$ et pH$_{KCl}$ (ou pH$_{CaCl2}$) extraites des bases de données BDAT (INRA-GisSol 2018) et NCSS (NCSS 2018) compilées (« filtrées » au sens d'Excel™) selon leurs agro-pédo-climat (APC ; EP2728353) ou encore plus simplement leur origine géographique en France. Par exemple, j'ai compilé 12 sous-ensembles (échantillons) de valeurs de pH et $\Delta$pH provenant de la Région Centre en France (BDAT ; INRA-GisSol 2018). Quelques 33 valeurs (n OBS, Tableau 1) ont été choisies aléatoirement (fonction *alea()* ; Excel™) 12 vois afin d'obtenir les valeurs moyennes au Tableau 1.

Pour ce qui est de la fonction [RES :: $\Delta$pHi]. Celle-ci est tracée, par exemple, à partir des données rapportées dans EP2730926 aux tableaux 1 et 2 (cf. tableau 1 - EP2730926 ; « *Dynamique métabolique, massique ou génomique (nga) de la fraction bactérienne du sol (BAC) pour le calcul de la première dérivée d'une fonction polynomiales de deuxième ou troisième degré au pHo (f'(pHo) = 0,00)*. », ou encore tableau 2 - EP2730926 ; « *Coefficients des équations polynomiales pH = aX$^3$ + bX$^2$ + cX+ d pourchacun des jeuxde données publiés au tableau* 1 »), et cela selon le schéma proposé ici à la Figure 2, sachant que les notions de courbes fixes et variables sont décrites clairement dans EP2730926. Encore une fois (cf. Figure 2), la mesure de la résilience est obtenue par superposition de la courbe de la dynamique bactérienne fonction de pH *in vitro* (courbe variable) à une autre courbe fixée horizontalement (courbe fixe) à partir d'un pH optimal (pHo) pour ladite dynamique bactérienne (ngaBAC).

La détermination graphique et mathématique d'iSAB est représentée à la Figure 3. Cette mise en relation de DEF et RES par rapport à ΔpHi implique ici la réalisation empirique des deux fonctions présentées aux Figures 1 et 2.

DEF et RES sont conjointement mis en relations par rapport à ΔpHi afin de déterminer iSAB = [DEF - RES]$_{ΔpHi}$. Tendanciellement, eAZB progresse selon iSAB (Figure 5) et peut donc servir d'indicateur élémentaire d'eAZB. En effet, la pression acidobasique provenant de la réalisation de l'acidité échangeable à travers la campagne sera donc d'autant plus faible que ΔpHi est important (Figure 4), et l'eAZB d'autant plus grande si cette pression ΔpH est importante puisque c'est dans de telles situations que les azotobactéries indigènes profitent le plus de la bioaugmentation de leurs populations résidsphériques, y compris mais pas exclusivement, par inoculation des RCS. Au contraire, les azotobactéries indigènes soumises à des ΔpHi de moindres amplitudes seront moins affectées et nécessiteront peu ou pas de telles augmentations. iSAB reflète, en amplifiant ΔpHi, l'opportunité d'augmenter les contingents d'azotobactéries résidusphériques en vue d'une augmentation d'eAZB (Figure 5). Ces deux fonctions de ΔpHi superposées, DEF et RES, a priori généralisables et applicables à l'ensemble des APC (EP2728353) tempérés d'Europe, définissent iSAB = [DEF - RES] fonctions de ΔpHi (Figure 4).

Ces relations DEF, RES et iSAB fonctions de ΔpHi seront raffinées lors du développement expérimental plus poussé de l'invention. Cela dit, à ce stade du développement expérimental, le paramétrage proposé au Tableau 2 permet néanmoins de réaliser l'invention correctement.

**Tableau 2** : Paramétrage des relations DEF, RES et iSAB (cf. Figures 3 et **4**) fonctions de ΔpHi ; les coefficients a$_i$ et b$_i$ (i = 1, 2 ou 3 pour DEF, RES et iSAB, respectivement) peuvent à ce stade appartenir à une gamme de valeurs +/- les écarts-types proposés entre (guillemets).

| Relation (Figures 2 et 3) | a$_i$ (écartypes) | b$_i$ (écartypes) |
|---|---|---|
| DEF = a$_1$ + b$_1$·ΔpHi | 1,80 (0,20) | -1,40 (0,25) |
| RES = a$_2$ + b$_2$·ΔpHi | 0,015 (0,012) | 0,340 (0,025) |
| iSAB = a$_3$ + b$_3$·ΔpHi | 1,80 (0,05) | -1,76 (0,04) |

## APPLICATION AGRONOMIQUE DE L'INVENTION

La relation tendancielle et partielle d'eAZB par rapport iSAB (Figure 5) ne préjudicie par l'utilisation de cette *indicateur élémentaire* (Lariez et al. 2015). L'agrégation de plusieurs indicateurs d'eAZB de ce type en un seul indice agrégé d'eAZB, iAZB (EP17196251.7) a été ici refaite avec iSAB au sens de la présente invention en remplacement de l'indicateur putatif « iRES » (EP2730926). En abîme de la Figure 7 (haut) la fonction [eAZB :: iSAB] (normalisée) décrivant cette augmentation tendancielle d'eAZBrdn selon iSAB rapportée aux Figure 5 et 6 ; *idem* pour eAZBrun (non rapportée). Notons qu'iSAB est strictement fonction de ΔpHi (Figure 4), sans pour autant être redondant. En effet, puisque ΔpHi permet de jumeler les deux variables, DEF et RES (Figure 3), c'est la pente de la relation [iSAB :: ΔpHi] (Figure 5) qui détermine la contribution de ΔpHi à DEF, et donc de iSAB à iAZB (Figure 7).

Enfin et sans surprise, la dose prévisionnelle d'engrais-N (dN) au sens de Claude 2017 et EP17196251.7 diminue progressivement selon l'augmentation d'iAZB ;

Le statut acidobasique du sol est maintenant partie intégrante d'iAZB, de dN et de la FAR (Claude 2017 ; EP17196251.7), ce qui n'était pas véritablement le cas avec EP2730926 simplement assimilé à ΔpH par Claude 2017 faute d'indicateurs élémentaires facilement réalisables sans manipulations relativement onéreuses. En ce sens, la présente invention reprend, résume et instrumentalise EP2730926.

L'invention est applicable aux populations bactériennes et/ou azotobactériennes du sol. Bien que l'ubiquité des azotobactéries (eg. Orr et al. 2011) fait que iSAB applicable aux premières soit aussi putativement applicable aux dernières, je propose néanmoins à la Figure 8 un projet de kit d'incubation permettant de déterminer simultanément les relations bimodales ngaBAC :: pH (*supra* Figure 2) pour les bactéries et les azotobactéries du sol. L'idée est de s'assurer que les conditions d'incubation des bactéries et azotobactéries soient (quasi) identiques. L'incorporation d'un puit plus petit avec milieux de culture azoté au sien d'un puit plus large avec milieux de culture sans azote permet de segmenter un même aliquote de la susdite fraction azotobactérienne du sol et d'obtenir *deux* cinétiques parfaitement commensurables mais différentes selon leurs activités diazotrophiques. A suivre.

## Références

Claude, P-Ph. 2017. Fertilisation raisonnée et azotobactérisation des résidus de culture. Poster no. 19, Thème 3 : Indicateurs, méthodes de raisonnement et OAD. 13ièmes Rencontres de la fertilisation raisonnée et de l'analyse.

8 & 9 novembre 2017, Cité, Centre de congrès de Nantes / Comifer et le Gemas - http://www.comifer.asso.fr/index.php/fr/evenements/rencontres-2017/posters-des-rencontres-2017.html

Claude, P-Ph. et L. Fillion. 2004. Effet de l'apport d'un Inoculum bactérien aux résidus de culture de maïsgrain au Sol sur le rendement et la qualité de blés d'hiver panifiables en France. Agrosolutions 15(1) : 23-29.

Colomb, B. 2017. Guide de la fertilisation raisonnée (2ième édition). Éditions France Agricole, 75493 Paris.

Edmeades, DC, CE Smart et DM Wheeler. 1983. Aluminium toxicity in New Zealand soils: Preliminary results on the development of diagnostic criteria, New Zealand J. Agric. Res., 26:4, 493-501

EP2730926 (Claude 2014) Diagnosis of the microbiological state of soils according to the resilience of its bacterial population. https://patents.google.com/patent/EP2730926A1/da

EP2728353 (Claude 2014) Method for assigning an agro/micro-pedoclimate (AMPC) to an agricultural plot, and for forming microbial consortia. https://patents.google.com/patent/EP2728353A1/da

EP2845906 (Claude 2015) Method for assessing the carbon efficiency of soil bacteria. https://patents.google.com/patent/EP2845906A1 /en

EP2942621 (Claude 2015) Diagnostic de l'état diazotrophe de sols arables et préconisation des apports d'engrais azoté. https://patents.google.com/patent/EP2942621A1/sv

Fernandez-Calvino, D. et Erland Baath. 2010. Growth response of the bacterial community to pH in soils differing in pH. FEMS Microbiol Ecol 73 (2010) 149-156

INRA GisSol 2018. Base de données d'analyses de terre (BDAT). http://webapps.aissol.fr/aeosol/

Lairez, J et al. 2015. Agriculture et Développement Durable ; guide pour l'évaluation multicritère. Eds. Quae 78026 Versailles Cedex).

Martyniuk, S et M Martyniuk. 2003. Occurrence of Azotobacter Spp. in Some Polish Soils. Polish Journal of EnvironmentalStudies Vol. 12, No. 3 (2003), 371-374

NCSS 2018. National Coopérative Soil Survey / National Coopérative Soil Survey Characterization Database / http://ncsslabdatamart.sc.egov.usda.gov/

Orr, CH, A James, C Leifert, JM Cooper et SP Cummings. 2011. Diversity and Activity of Free-Living Nitrogen-Fixing Bacteria and Total Bacteria in Organic and Conventionally Managed Soils. Appl. Environ. Mircrobiol. p. 911-919 Vol. 77, No. 3

Wonnacott, TH et RJ Wonnacott. 1991 (traduction française). Statistique - économie, gestion, sciences, médecine (4ième édition). Economia, 49, rue Héricart, 75015 Paris.

**Revendications**

1. **Méthode de fertilisation raisonnée adaptée à l'azotobactérisation (AZB) des résidus de culture cellulosiques au sol (RCS) et de cultures intermédiaires pièges à nitrates (cipan)** comportant la formation d'un indicateur élémentaire de la résilience de la fraction bactérienne de la microflore d'un sol arable en termes de diversité, d'activité et/ou d'abondance lorsque confrontée à des variations de pH du sol environnant **caractérisée en ce que** la formation de l'indicateur élémentaire, iSAB (*indicateur élémentaire* de la résilience acidobasique des azotobactéries indigènes telluriques défiées par $\Delta$pH), est obtenu directement à l'aide de relations préétablies, à savoir ;

   • une première relation [$\Delta$pH :: pH] représentant quantitativement la *défiance* acidobasique du sol, DEF (*défiance* acidobasique d'un sol à l'égards de populations azotobactériennes qu'il abrite) définie formellement comme le ratio entre l'amplitude de l'acidité échangeable ($\Delta$pHo - $\Delta$pHn) et le $\Delta$pH associée au pHo ($\Delta$pHo) et obtenue par la dérivée première égale à 0,00 de la relation [$\Delta$pH :: pH] sur la base de valeurs pH du sol, avantageusement

le $pH_{eau}$ afin d'assurer un $\Delta pH$ (*alias* acidité échangeable du sol attribuable à l'aluminium et l'hydrogène adsorbés sur le complexe d'échange du sol mais échangeable par d'autres cations tels que le $K^+$ et/ou le $Ca^{++}$) mesurable ($\Delta pHi$ ; $\Delta pH$ moyen d'un ensemble de $\Delta pH$ sur la base de la différence $pH_{eau}$ - $pH_{KCl}$ (ou $pH_{CaCl2}$) mesuré par l'utilisateur) prélevées à travers le temps ou résultant de différentes pratiques culturales ou provenant de variations spatiales répertoriées dans diverses bases de données existantes, sachant que $\Delta pHo$ est le $\Delta pH$ optimal correspondant à la première dérivée nulle (0,00) de la relation [$\Delta pH :: pH$] et $\Delta pHn$ le $\Delta pH$ minimum d'un ensemble de $\Delta pH$,

• une deuxième relation [ngaBAC :: pH] représentant quantitativement la *résilience* acidobasique des azotobactéries indigènes du sol, RES (résilience acidobasique des azotobactéries indigènes du sol confrontées à des variations de pH à hauteur de $\Delta pH$ = [$pH_{eau}$ - $pH_{KCl}$] voire [$pH_{eau}$ - $pH_{CaCl2}$]) définie comme le ratio entre le $\Delta pH$ effectif ($\Delta pHe$) et $\Delta pHi$, $\Delta pHe$ étant repérable dès que ngaBAC (*n*ombre de cellules bactériennes (n), leurs masses (*g*) et/ou leurs abondances relative à celles des autres fractions de la microflore du sol, leurs diversité génomique et/ou phénotypiques et/ou leurs *a*ctivités métaboliques et/ou cataboliques) est appréciablement affecté par $\Delta pH$ lorsque le minimum d'une certaine *l'intervalle de confiance* (ic) de la susdite courbe fixe croise (intersecte) le maximum de l'ic de la susdite courbe variable au point « pi »,

• la superposition, graphique ou mathématique, par rapport à $\Delta pHi$ = [$pH_{eau}$ - $pH_{KCl}$], de ces deux relations - DEF et RES, afin d'obtenir la différence iSAB = [DEF - RES] à titre d'indicateur élémentaire du degré de résilience acidobasique des populations azotobactériennes indigènes du sol,

• et enfin l'intégration d'iSAB dans un indice agrégé d'eAZB servant au calcul des doses prévisionnelles d'engrais azotés (dN) adaptées au fonctionnement de telles azotobactérisation des résidus de culture cellulosiques en fertilisation raisonnée.

2. **Méthode de fertilisation raisonnée adaptée à l'azotobactérisation (AZB) des résidus de culture cellulosiques au sol (RCS) et de cultures intermédiaires pièges à nitrates (cipan)** selon la revendication 1 **caractérisée en ce que** la défiance acidobasique du sol, DEF, est exprimée par rapport à $\Delta pHi$ selon la relation DEF = $a_1$ + $b_1 \cdot \Delta pHi$.

3. **Méthode de fertilisation raisonnée adaptée à l'azotobactérisation (AZB) des résidus de culture cellulosiques au sol (RCS) et de cultures intermédiaires pièges à nitrates (cipan)** selon la revendication 2 **caractérisée en ce que** les coefficients $a_1$ et $b_1$ peuvent appartenir à une gamme de valeurs comprises entre 1,60 et 2,00 pour $a_1$ et -1,15 et -1,65 pour $b_1$.

4. **Méthode de fertilisation raisonnée adaptée à l'azotobactérisation (AZB) des résidus de culture cellulosiques au sol (RCS) et de cultures intermédiaires pièges à nitrates (cipan)** selon l'une quelconque des revendications précédentes **caractérisée en ce que** pour la deuxième relation, [ngaBAC :: pH] représentant quantitativement la *résilience* acidobasique des azotobactéries indigènes du sol, RES, lesdites intervalles de confiance (ic) sont fonctions des variations des valeurs de pH et de la dynamique des population azotobactérienne (ngaBAC) correspondante selon l'équation ;

$$ic = (a + bX_o) +/- t_{0,025} \cdot s \cdot racine(1/n + [(X_o - \dot{X})^2 / \sum X^2])$$

lorsque que ($a + bX_o$) décrit empiriquement la progression d'ngaBAC selon $X_o$ = pH à partir de pHo, $t_{0,025}$ est la valeur critique de la statistique t lorsque les ddl (degrés de liberté) = nombre d'observations moins 1, s est *l'écart-type* et $(X_o - \dot{X})^2$ la somme des carrés des différences entre $X_o$ et la moyenne X pour les n observations.

5. **Méthode de fertilisation raisonnée adaptée à l'azotobactérisation (AZB) des résidus de culture cellulosiques au sol (RCS) et de cultures intermédiaires pièges à nitrates (cipan)** selon la revendication 4 **caractérisée en ce que** la résilience acidobasique des azotobactéries du sol, RES, est exprimée par rapport à $\Delta pHi$ selon la relation RES = $a_2$ + $b_2 \cdot \Delta pHi$.

6. **Méthode de fertilisation raisonnée adaptée à l'azotobactérisation (AZB) des résidus de culture cellulosiques au sol (RCS) et de cultures intermédiaires pièges à nitrates (cipan)** selon la revendication 5 **caractérisée en ce que** les coefficients $a_2$ et $b_2$ peuvent appartenir à une gamme de valeurs comprises entre 0,003 et 0,027 pour $a_2$ et 0,315 et 0,365 pour b2.

7. **Méthode de fertilisation raisonnée adaptée à l'azotobactérisation (AZB) des résidus de culture cellulosiques au sol (RCS) et de cultures intermédiaires pièges à nitrates (cipan)** selon l'une quelconque des revendications précédentes **caractérisée en ce que** la superposition, graphique ou mathématique, par rapport à $\Delta pHi$ = [$pH_{eau}$ -

$pH_{KCl}$], de ces deux relations - DEF et RES, afin d'obtenir la différence iSAB = [DEF - RES] à titre d'indicateur élémentaire du degré de résilience acidobasique des populations azotobactériennes indigènes du sol donne lieu à une famille de relations iSAB fonctions de $\Delta pHi$ selon le type de situations agronomiques, types de sols, localisation géographique de la parcelle ou toutes autres caractéristiques agro-pédo-climatiques pouvant affecter le statut acidobasique (SAB) du sol.

8. **Méthode de fertilisation raisonnée adaptée à l'azotobactérisation (AZB) des résidus de culture cellulosiques au sol (RCS) et de cultures intermédiaires pièges à nitrates (cipan)** selon la revendication 7 **caractérisée en ce que** l'indicateur du degré de résilience acidobasique des populations azotobactériennes indigènes du sol, iSAB, est exprimé par rapport à $\Delta pHi$ selon la relation iSAB = $a_3 + b_3 \cdot \Delta pHi$.

9. **Méthode de fertilisation raisonnée adaptée à l'azotobactérisation (AZB) des résidus de culture cellulosiques au sol (RCS) et de cultures intermédiaires pièges à nitrates (cipan)** selon la revendication 8 **caractérisée en ce que** les coefficients $a_3$ et $b_3$ peuvent appartenir à une gamme de valeurs comprises entre 1,75 et 1,85 pour $a_3$ et -1,72 et -1,80 pour $b_3$.

10. **Méthode de fertilisation raisonnée adaptée à l'azotobactérisation (AZB) des résidus de culture cellulosiques au sol (RCS) et de cultures intermédiaires pièges à nitrates (cipan)** selon l'une

quelconque des revendications précédentes **caractérisée en ce que** l'intégration d'iSAB dans un indice agrégé d'eAZB (efficacité relative en termes de *rendement protéique* (RDN ; $kg\text{-}N_{grain}$/ha) ou de *rendement unitaire* (RUN ; $kg\text{-}N_{grain}$/uN) de l'azotobactérisation des résidus de culture (cellulosiques) au sol (RCS) par rapport à un témoin non azotobactérisés) servant au calcul des doses prévisionnelles d'engrais azotés (dN) adaptées à l'azotobactérisation des résidus de culture ce fait comme suit ;

iAZB = $\Sigma(Si \times Pi)$ pour les i = 1, 2, 3 ... $\to$ nombre d'indicateurs (*n*), y compris iSAB *Si* étant ici le score normalisé (base 100 ou 1,00) de l'indicateur élémentaire affectant eAZB, et *Pi* le poids (0,00 à 1,00) attribué à ce score, pAZB est ici en sorte la contribution (fourniture) en N des microorganismes phytogènes du sol, y compris ceux réintroduits par l'inoculation, et **en ce que** dN fonction de l'indicateur agrégé, iAZB, obtenu par agrégation des indicateurs élémentaires d'eAZB est calculé comme suite ;

$$dN = a \times pRDT - pAZB$$

sachant que ;

- a = $b_{ARVALIS}$ / $eAZB_{RUN}$
- pAZB = pRDN - pRDN/eAZBRDN

et que pRDT et pRDN sont les objectifs, ou potentiels, de rendements agronomiques ($Q_{Xgrain}$/ha) et protéiques ($kg\text{-}N_{grain}$/ha), $eAZB_{RDN}$ et $eAZB_{RUN}$ les valeurs d'eAZB en termes de RDN et RUN (rendements unitaires ; $kg\text{-}N_{grain}$ ou $kg\text{-}N_{biomasse}$ /unité d'N fertilisant), respectivement et par rapport à un témoin non traité, et que a est le coefficient b dit d'Arvalis™ ($b_{ARVALIS}$), alias « besoin unitaire » (i.e. unités d'N par $Qx_{grain}$), divisée par $eAZB_{RUN}$.

**Patentansprüche**

1. **Methode der systematischen Düngung, angepasst an die Azotobakterisierung (AZB) von Rückständen von Zellulosepflanzen (RCS) im Boden und nitratbindende Zwischenkulturen (Cipan),** einschließlich der Bildung eines elementaren Indikators für die Widerstandsfähigkeit der bakteriellen Fraktion der Mikroflora eines Ackerbodens in Bezug auf Diversität, Aktivität und/oder Abundanz, wenn diese mit Schwankungen des pH-Werts des umgebenden Bodens konfrontiert wird, **dadurch gekennzeichnet, dass** die Bildung des elementaren Indikators, iSAB (*elementarer Indikator* für die Säure-Basen-Resilienz einheimischer Boden-Azotobakterien,die durch $\Delta pH$ konfrontiert sind) direkt mithilfe vorher festgelegter Beziehungen erzielt wird, nämlich;

- eine erste Beziehung [$\Delta pH :: pH$], die quantitativ den *Säure-Basen-Stress* des Bodens darstellt, DEF (Säure-Basen-Stress eines Bodens, als die Belastung der Azotobakterien-Populationen durch Säure-Basen-Veränderungen im Boden, der sie beherbergt), formal definiert als das Verhältnis zwischen der Amplitude der veränderlichen Acidität (ΔpHo - ΔpHn) und der ΔpH des pHo (ΔpHo) und wird erhalten durch die erste Ableitung gleich 0,00 des Verhältnisses [ΔpH :: pH] auf der Grundlage von pH-Werten des Bodens, vorteilhafterweise dem $pH_{Wasser}$, um eine ΔpH (*alias* veränderliche Acidität des Bodens, die dem an den Bodenaustauschkomplex adsorbierten Aluminium und Wasserstoff zuzuschreiben ist, aber austauschbar durch andere Kationen wie $K^+$ und/oder $Ca^{++}$) sicherzustellen, die messbar ist (ΔpHi ;ΔpH-Mittelwert eines Satzes von ΔpH auf der Grundlage der Differenz $pH_{wasser}$ - $pH_{KCl}$ (oder $PH_{CaCl2}$) vom Benutzer gemessen), die über den Verlauf der Zeit beprobt wurden, oder die aus unterschiedlichen Anbaumethoden resultieren oder von räumlichen Variationen herrühren die in verschiedenen bestehenden Datenbanken aufgelistet sind, wobei ΔpHo der optimale ΔpH ist, der der ersten Nullableitung (0,00) des Verhältnisses [ΔpH :: pH] entspricht, und ΔpHn der minimale ΔpH einer Menge von ΔpH ist,

• eine zweite Beziehung [ngaBAC:: pH], die die *Säure-Basen-Resistenz* einheimischer Bodenazotobakterien quantitativ darstellt, RES (Säure-Basen-Resistenz einheimischer Bodenazotobakterien, die mit pH-Schwankungen in der Höhe von ΔpH = [$pH_{Wasser}$ - $pH_{KCl}$] bzw. [$pH_{wasser}$ - $pH_{CaCl2}$] konfrontiert sind), definiert als das Verhältnis zwischen dem effektiven ΔpH (ΔpHe) und ΔpHi, wobei ΔpHe findbar ist, sobald ngaBAC (Anzahl der Bakterienzellen (*n*), ihre Massen (*g*) und/oder ihre relative Häufigkeit im Vergleich zu den anderen Fraktionen der Bodenmikroflora, ihre genomische und/oder phänotypische Diversität und/oder ihre metabolischen und/oder katabolischen Aktivitäten (*a*)) signifikant von ΔpH beeinflusst ist, wenn das Minimum eines bestimmten *Konfidenzintervalls* (ic) der oben genannten festen Kurve das Maximum des ic der oben genannten variablen Kurve am Punkt "pi" kreuzt (schneidet),

• die grafische oder mathematische Überlagerung, in Bezug auf ΔpHi = [$pH_{eau}$ - $pH_{KCl}$], dieser zwei Beziehungen - DEF und RES -, um die Differenz iSAB = [DEF - RES] als elementaren Indikator für den Grad der Säure-Basen-Resilienz der einheimischen azotobakteriellen Bodenpopulationen zu erhalten,

• und schließlich die Integration von iSAB in einen aggregierten Index von eAZB, der zur Berechnung der voraussichtlichen Stickstoffdüngerdosen (dN) dient, die an das Funktionieren solcher Azotobakterisierung von Resten von Zellulosepflanzen bei durchdachter Düngung angepasst sind.

2. **Methode der systematischen Düngung, angepasst an die Azotobakterisierung (AZB) von Rückständen von Zellulosepflanzen (RCS) im Boden und nitratbindende Zwischenkulturen (Cipan),** gemäß Anspruch 1 **dadurch gekennzeichnet, dass** der Säure-Basen-Stress des Bodens, DEF, im Verhältnis zu ΔpHi gemäß der Beziehung DEF = a1 + b1·ΔpHi ausgedrückt wird.

3. **Methode der systematischen Düngung, angepasst an die Azotobakterisierung (AZB) von Rückständen von Zellulosepflanzen (RCS) im Boden und nitratbindende Zwischenkulturen (Cipan),** gemäß Anspruch 2 **dadurch gekennzeichnet, dass** die Koeffizienten $a_1$ und $b_1$ einem Wertebereich zwischen 1,60 und 2,00 für $a_1$ und -1,15 und -1,65 für $b_1$ angehören können.

4. **Methode der systematischen Düngung, angepasst an die Azotobakterisierung (AZB) von Rückständen von Zellulosepflanzen (RCS) im Boden und nitratbindende Zwischenkulturen (Cipan),** gemäß einem der vorhergehenden Ansprüche, **dadurch gekennzeichnet, dass** für das zweite Verhältnis [ngaBAC: pH], das die *Säure-Basen-Resilienz,* RES, der im Boden heimischen Azotobakterien quantitativ darstellt, die Konfidenzintervalle (ic) Funktionen der Veränderungen der pH-Werte und der entsprechenden azotobakteriellen Populationsdynamik (ngaBAC) sind, gemäß der Gleichung;

$$ic = (a + bX_o) +/- t_{0,025} \cdot s \cdot \text{Wurzel}(1/n + [(X_o - \dot{X})2/ \sum X2])$$

wenn (a + $bX_o$) empirisch den Verlauf von ngaBAC gemäß $X_o$ = pH ab pHo beschreibt, $t_{0,025}$ ist der kritische Wert der t-Statistik, wenn die ddl (Freiheitsgrade) = Anzahl der Beobachtungen minus 1, $s$ ist die *Standardabweichung* und $(X_o - X)^2$ ist die Summe der Quadrate der Differenzierungen zwischen $X_o$ und dem Mittelwert X für die n Beobachtungen.

5. **Methode der systematischen Düngung, angepasst an die Azotobakterisierung (AZB) von Rückständen von Zellulosepflanzen (RCS) im Boden und nitratbindende Zwischenkulturen (Cipan),** gemäß Anspruch 4 **dadurch gekennzeichnet, dass** die Säure-Basen-Resilienz der Bodenazotobakterien, RES, in Bezug auf ΔpHi gemäß der Beziehung RES = $a_2$ + $b_2$·ΔpHi ausgedrückt wird.

6. **Methode der systematischen Düngung, angepasst an die Azotobakterisierung (AZB) von Rückständen von Zellulosepflanzen (RCS) im Boden und nitratbindende Zwischenkulturen (Cipan),** gemäß Anspruch 5 **dadurch**

**gekennzeichnet, dass** die Koeffizienten $a_2$ und $b_2$ einem Wertebereich zwischen 0,003 und 0,027 für $a_2$ und 0,315 und 0,365 für $b_2$ angehören können.

7. **Methode der systematischen Düngung, angepasst an die Azotobakterisierung (AZB) von Rückständen von Zellulosepflanzen (RCS) im Boden und nitratbindende Zwischenkulturen (Cipan),** gemäß einem der vorhergehenden Ansprüche, **dadurch gekennzeichnet, dass** die grafische oder mathematische Überlagerung dieser beiden Beziehungen - DEF und RES - in Bezug auf $\Delta pHi = [pH_{Wasser} - pH_{KCl}]$, um die Differenz iSAB = [DEF - RES] als elementaren Indikator für den Grad der Säure-Basen-Resilienz der einheimischen azotobakteriellen Bodenpopulationen zu erhalten, zu einer Familie von iSAB-Verhältnissen führt, die Funktionen von $\Delta pHi$ sind, je nach Art der agronomischen Situationen, Bodentypen, geografischen Lage des Grundstücks oder allen anderen agro-pedoklimatischen Merkmalen, die den Säure-Basen-Status (SAB) des Bodens beeinflussen können.

8. **Methode der systematischen Düngung, angepasst an die Azotobakterisierung (AZB) von Rückständen von Zellulosepflanzen (RCS) im Boden und nitratbindende Zwischenkulturen (Cipan),** gemäß Anspruch 7 **dadurch gekennzeichnet, dass** der Indikator für den Grad der Säure-Basen-Resilienz der einheimischen azotobakteriellen Bodenpopulationen, iSAB, in Bezug auf $\Delta pHi$ gemäß der Beziehung iSAB = $a_3 + b_3 \cdot \Delta pHi$ ausgedrückt wird.

9. **Methode der systematischen Düngung, angepasst an die Azotobakterisierung (AZB) von Rückständen von Zellulosepflanzen (RCS) im Boden und nitratbindende Zwischenkulturen (Cipan),** gemäß Anspruch 5 **dadurch gekennzeichnet, dass** die Koeffizienten $a_3$ und $b_3$ zu einem Wertebereich zwischen 1,75 und 1,85 für $a_3$ und zwischen -1,72 und -1,80 für $b_3$ gehören können.

10. **Methode der systematischen Düngung, angepasst an die Azotobakterisierung (AZB) von Rückständen von Zellulosepflanzen (RCS) im Boden und nitratbindende Zwischenkulturen (Cipan),** gemäß einem der vorhergehenden Ansprüche, **dadurch gekennzeichnet, dass** die Einbeziehung von iSAB in einen aggregierten Index von eAZB (relative Effizienz in Bezug auf den *Proteinertrag* (RDN; kg-$N_{Korn}$/ha) oderden *Einheitsertrag* (RUN; kg-$N_{Korn}$/uN) der Azotobakterisierung (AZB) von (zellulosehaltigen) Kultur-Rückständen im Boden (RCS) im Vergleich zu einer nicht azotobakterisierten Kontrollprobe), die zur Berechnung der an die Azotobakterisierung der Kulturrückstände angepasstenvoraussichtlichen Stickstoffdüngerdosen (dN)dient, wie folgt;

$$iAZB = \sum(Si \times Pi) \text{ für } i = 1, 2, 3 ... \rightarrow \text{Anzahl der Indikatoren}(n), \text{ und einschließlich iSAB}$$

wobei *Si* hier die normalisierte Punktzahl (Basis 100 oder 1,00) des elementaren Indikators ist, der eAZB beeinflusst, und *Pi* das Gewicht (0,00 bis 1,00) ist, das dieser Punktzahl zugewiesen wird, pAZB ist hier in Art der Beitrag (Bereitstellung) an N der phytogenen Mikroorganismen des Bodens, einschließlich der durch die Inokulation wiedereingeführten, und somit ist dN, eine Funktion des aggregierten Indikators, iAZB, der durch Aggregation der elementaren Indikatoren von eAZB erhalten wird, er wird wie folgt berechnet;

$$dN = a \times pRDT - pAZB$$

wobei gilt, dass;

- 

$$a = bARVALIS / eAZBRUN$$

- 

$$pAZB = pRDN - pRDN/eAZB_{RDN}$$

und dass pRDT und pRDN die Ziele oder Potenziale für agronomische Erträge ($Qx_{Korn}$/ha) und Proteinerträge (kg-$N_{Korn}$/ha) sind, $eAZB_{RDN}$ und $eAZB_{RUN}$ sind die Werte von eAZB in Bezug auf RDN und RUN (Einheitserträge; kg-$N_{Korn}$ oder kg-$N_{biomasse}$ / N-Dünger-Einheit), jeweils und im Vergleich zu einer unbehandelten Kontrollprobe, und a ist der sogenannte Arvalis ™ -Koeffizient b ($b_{ARVALIS}$) alias "Einheitsbedarf" (d. h. N-Einheiten pro $Qx_{Korn}$), geteilt durch $eAZB_{RUN}$.

**Claims**

1. **Method of integrated fertilizer management adapted to the azotobacterial treatment (AZB) of soil borne cellulosic crop residues (RCS) and catch crops (cipan)** comprising the formation of an elementary indicator of the resilience of the bacterial fraction of the microflora of an arable soil in terms of diversity, activity and/or abundance when confronted with variations in the pH of the surrounding soil **characterized in that** the formation of the indicator elementary, iSAB (*elementary indicator* of the acid-base resilience of indigenous telluric azotobacteria challenged by $\Delta$pH), is obtained directly using pre-established relationships, namely;

   • a first relationship [$\Delta$pH :: pH] quantitatively representing the acid-base *defiance* of the soil, DEF (acid-base-*defiance* of a soil with regard to azotobacterial populations it shelters) formally defined as the ratio between the amplitude of the exchangeable acidity ($\Delta$pHo - $\Delta$pHn) and the $\Delta$pH associated with the pHo ($\Delta$pHo) and obtained by the first derivative equal to 0.00 of the relation [$\Delta$pH :: pH] on the basis of soil pH values, preferably the water pH to ensure a $\Delta$pH (*aka* exchangeable acidity of the soil attributable to aluminum and hydrogen adsorbed on the soil exchange complex but exchangeable by other cations such as $K^+$ and / or $Ca^{++}$) measurable ($\Delta$pHi; $\Delta$pH average of a set of $\Delta$pH based on the difference $pH_{water}$ - $pH_{KCl}$ (or $pH_{CaCl2}$) measured by the user) taken over time or resulting from different cultural practices or from spatial variations listed in various existing databases, knowing that $\Delta$pHo is the optimal $\Delta$pH corresponding to the first zero derivative (0,00) of the relationship [$\Delta$pH :: pH] and $\Delta$pHn the minimum $\Delta$pH of a set of $\Delta$pH,
   • a second relationship [ngaBAC :: pH] quantitatively representing the acid-base *resilience* of the native soil azotobacteria, RES(acid-base resilience of native soil azotobacteria faced with pH variations up to $\Delta$pH = [$pH_{water}$ - $pH_{KCl}$] or even [$pH_{water}$ - $pH_{CaCl2}$]) defined as the ratio between the effective $\Delta$pH ($\Delta$pHe) and $\Delta$pHi, $\Delta$pHe being detectable as soon as ngaBAC (number of bacterial cells (***n***), their masses (g) and/or their abundances relative to those of other fractions of the soil microflora, their genomic and / or phenotypic diversity and/or their metabolic and/or catabolic activities) is appreciably affected by $\Delta$pH when the minimum of a certain confidence interval (ic) of the aforementioned fixed curve crosses (intersects) the maximum of the ic of the aforementioned variable curve at the point "pi",
   • the superposition, graphical or mathematical, with respect to $\Delta$pHi = [$pH_{water}$ - $pH_{KCl}$], of these two relationships - DEF and RES, to obtain the difference iSAB = [DEF - RES] as an elementary indicator of the degree of acid-base resilience of azotobacterial populations native to the soil,
   • and finally, the integration of iSAB into an aggregate index of eAZB used to calculate the forecast doses of nitrogen fertilizers (dN) adapted to the functioning of such azotobacterial treatment of cellulosic crop residues in integrated fertilizer management.

2. **Method of integrated fertilizer management adapted to the azotobacterial treatment (AZB) of soil borne cellulosic crop residues (RCS) and catch crops (cipan)** according to claim 1 **characterized in that** the acid-base defiance of the soil, DEF, is expressed with respect to $\Delta$pHi according to the relationship DEF = $a_1$ + $b_1 \cdot \Delta$pHi.

3. **Method of integrated fertilizer management adapted to the azotobacterial treatment (AZB) of soil borne cellulosic crop residues (RCS) and catch crops (cipan)** according to claim 2 **characterized in that** the coefficients $a_1$ and $b_1$ may belong to a range of values between 1.60 and 2.00 for $a_1$ and -1.15 and -1.65 for $b_1$.

4. **Method of integrated fertilizer management adapted to the azotobacterial treatment (AZB) of soil borne cellulosic crop residues (RCS) and catch crops (cipan)** according to any one of the preceding claims **characterized in that** for the second relation, [ngaBAC :: pH] quantitatively representing acid-base resilience of the soil's native azotobacteria, RES, the said confidence intervals (ic) are a function of variations in pH values and the corresponding azotobacterial population dynamics (ngaBAC) according to the equation;

$$ic = (a + bX_o) +/- t_{0.025} \cdot s \cdot \text{root}(1/n + [(X_o - \dot{X})^2/\sum X^2])$$

when (a + $bX_o$) empirically describes the progression of ngaBAC according to $X_o$ = pH from pHo, $t_{0.025}$ is the critical value of the statistic t when the ddl (degrees of freedom) = number of observations minus 1, s is the *standard deviation* and $(X_o - \dot{X})^2$ the sum of the squares of the differences between $X_o$ and the mean $\dot{X}$ of the n observations.

5. **Method of integrated fertilizer management adapted to the azotobacterial treatment (AZB) of soil borne cellulosic crop residues (RCS) and catch crops (cipan)** according to claim 4 **characterized in that** the acido-base resilience of the soil's azotobacteria, RES, is expressed with respect to $\Delta$pHi according to the relationship RES $= a_2 + b_2 \cdot \Delta$pHi.

6. **Method of integrated fertilizer management adapted to the azotobacterial treatment (AZB) of soil borne cellulosic crop residues (RCS) and catch crops (cipan)** according to claim 5 **characterized in that** the coefficients $a_2$ and $b_2$ may belong to a range of values between 0.003 and 0.027 for $a_2$ and 0.315 and 0.365 for $b_2$.

7. **Method of integrated fertilizer management adapted to the azotobacterial treatment (AZB) of soil borne cellulosic crop residues (RCS) and catch crops (cipan)** according to any one of the preceding claims **characterized in that** the superposition, graphic or mathematical, with respect to $\Delta$pHi = [$pH_{water}$ - $pH_{KCl}$], of these two relationships - DEF and RES, in order to obtain the difference iSAB = [DEF - RES] as an elementary indicator of the degree of acid-base resilience of indigenous azotobacterial populations of the soil gives rise to a family of iSAB relations functions of $\Delta$pHi according to the type of agronomic situations, soil types, geographical location of the plot or any other agro-pedo-climatic characteristics that may affect the acid-base status (SAB) of the soil.

8. **Method of integrated fertilizer management adapted to the azotobacterial treatment (AZB) of soil borne cellulosic crop residues (RCS) and catch crops (cipan)** according to claim 7 **characterized in that** the indicator of the degree of acid-base resilience of native azotobacterial populations of the soil, iSAB, is expressed in relation to $\Delta$pHi according to the relationship iSAB = $a_3 + b_3 \cdot \Delta$pHi.

9. **Method of integrated fertilizer management adapted to the azotobacterial treatment (AZB) of soil borne cellulosic crop residues (RCS) and catch crops (cipan)** according to claim 8 **characterized in that** the coefficients $a_3$ and $b_3$ may belong to a range of values between 1.75 and 1.85 for $a_3$ and -1.72 and -1.80 for $b_3$.

10. **Method of integrated fertilizer management adapted to the azotobacterial treatment (AZB) of soil borne cellulosic crop residues (RCS) and catch crops (cipan)** according to any one of the preceding claims **characterized in that** the integration of iSAB in an aggregate index of eAZB (relative efficiency in terms of protein *yield* (RDN; kg-$N_{grain}$/ha) or unit *yield* (RUN; kg-N $_{grain}$/uN) of the azotobacteriation of crop residues (cellulosic) in the soil (RCS) compared to a non-azotobacteria control)used to calculate the forecast doses of nitrogen fertilizers (dN) adapted to the azotobacteriation of crop residues this fact as follows;

$$\text{iAZB} = \sum(\text{Si x Pi}) \text{ for i = 1, 2, 3 ...} \rightarrow \text{number of indicators } (n), \text{ including iSAB}$$

*Si* here being the normalized score (base 100 or 1.00) of the elementary indicator affecting eAZB, and *Pi* the weight (0.00 to 1.00) attributed to this score, pAZB in this case the contribution (supply) in N of the phytogenic microorganisms of the soil, including those reintroduced by inoculation, and **in that** dN function of the aggregated indicator, iAZB, obtained by aggregation of the elementary indicators of eAZB is calculated as follows;

$$\text{dN} = \text{a x pRDT - pAZB}$$

knowing that;

- $$\text{a} = \text{b}_{ARVALIS} / \text{eAZB}_{RUN}$$

- $$\text{pAZB} = \text{pRDN - pRDN/eAZB}_{RDN}$$

and that pRDT and pRDN are respectively the target, or potential, agronomic (Qx$_{grain}$/ha) and protein yields (kg-$N_{grain}$/ha), eAZB$_{RDN}$ and eAZB$_{RUN}$ the values of eAZB in terms of RDN and RUN (unit yields; kg-$N_{grain}$ or kg-$N_{biomass}$ /unit of N fertilizing), respectively and compared to an untreated control, and that a is the coefficient b called Arvalis $^{™}$ ($b_{ARVALIS}$ ), or requirement, (i.e. "units" or kg of fertilizer N required per $Qx_{grain}$), divided by $eAZB_{RUN}$.

$$DEF = [\Delta pHo - \Delta pHn] / \Delta pHo$$

**Figure 1**

$$RES = [\Delta pHe / \Delta pHi]$$

Figure 2

**Figure 3**

Figure 4

Figure 5

**Figure 6**

Figure 7

(a)

(b)

(c)

(d)

(e)

(f)

Figure 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2730926 A
- EP 17196251
- EP 2728353 A
- EP 2845906 A
- EP 2942621 A
- EP 2730926 A1
- EP 2728353 A1
- EP 2845906 A1
- EP 2942621 A1

**Littérature non-brevet citée dans la description**

- **CLAUDE, P-PH.** Thème 3 : Indicateurs, méthodes de raisonnement et OAD. 13ièmes Rencontres de la fertilisation raisonnée et de l'analyse. *Fertilisation raisonnée et azotobactérisation des résidus de culture,* 2017, http://www.comifer.asso.fr/index.php/fr/evenements/rencontres-2017/posters-des-rencontres-2017.html
- **CLAUDE, P-PH. ; L. FILLION.** Effet de l'apport d'un Inoculum bactérien aux résidus de culture de maïsgrain au Sol sur le rendement et la qualité de blés d'hiver panifiables en France. *Agrosolutions,* 2004, vol. 15 (1), 23-29
- **COLOMB, B.** Guide de la fertilisation raisonnée. Éditions France Agricole, 75493 Paris, 2017
- **EDMEADES, DC ; CE SMART ; DM WHEELER.** Aluminium toxicity in New Zealand soils: Preliminary results on the development of diagnostic criteria. *New Zealand J. Agric. Res.,* 1983, vol. 26 (4), 493-501
- **FERNANDEZ-CALVINO, D ; ERLAND BAATH.** Growth response of the bacterial community to pH in soils differing in pH. *FEMS Microbiol Ecol,* 2010, vol. 73, 149-156
- *Base de données d'analyses de terre (BDAT),* 2018, http://webapps.aissol.fr/aeosol/
- guide pour l'évaluation multicritère. **LAIREZ, J et al.** Agriculture et Développement Durable. Eds. Quae 78026 Versailles Cedex, 2015
- **MARTYNIUK, S ; M MARTYNIUK.** Occurrence of Azotobacter Spp. in Some Polish Soils. *Polish Journal of EnvironmentalStudies,* 2003, vol. 12 (3), 371-374
- *National Coopérative Soil Survey / National Coopérative Soil Survey Characterization Database,* 2018, http:/ncsslabdatamart.sc.egov.usda.gov
- **ORR, CH ; A JAMES ; C LEIFERT ; JM COOPER ; SP CUMMINGS.** Diversity and Activity of Free-Living Nitrogen-Fixing Bacteria and Total Bacteria in Organic and Conventionally Managed Soils. *Appl. Environ. Mircrobiol.,* 2011, vol. 77 (3), 911-919
- Statistique - économie, gestion, sciences, médecine. **WONNACOTT, TH ; RJ WONNACOTT.** Economia. 49, rue Héricart, 75015 Paris, 1991